# EUROPEAN PATENT SPECIFICATION

(11) **EP 3 586 806 B1**
(45) Date of publication and mention of the grant of the patent: **03.11.2021**
(21) Application number: 19174148.7
(22) Date of filing: 13.05.2019
(51) Int. Cl.: A61F 13/49, A61F 13/84

(54) **SANITARY ARTICLES FOR ABSORBING ODOUR**
HYGIENISCHE ARTIKEL UM GERUCH ZU ABSORBIEREN
ARTICLES SANITAIRES POUR ABSORBER LES ODEURS

(30) Priority: 21.06.2018 SE 1850764
(43) Date of publication of application: 01.01.2020
(73) Proprietor: Sanicare AB, 311 23 Falkenberg (SE)
(72) Inventor: ULFSTRÖM, Cenneth, 311 40 Falkenberg (SE); BLACKBURN, Andrew, Winchester, Hampshire, SO23 0QW (GB)
(74) Representative: Bergenstråhle & Partners AB

(56) References cited:
- EP-A1- 0 510 619
- WO-A1-2015/094068
- GB-A- 2 308 303
- US-A- 5 154 960
- US-A1- 2018 064 585

## Description

### Technical field

The present invention relates generally to sanitary articles comprising an odour-absorbent layer intended to be arranged in the crotch region of a human being, the layer being arranged for absorbing odour originating from the human being.

### Background art

To use odour prevention- or odour absorbing products in absorbing sanitary articles, such as diapers, sanitary napkins and sanitary pads is an important comfort factor for the user of the sanitary article. Body fluids, such as urine and menstruation blood, and excrements may be collected and stored in the absorbent sanitary article. Hereby odours may arise. It is important for the wearer that these odours do not spread into the environment.

A current odour-absorbent sanitary article of the applicant called "Sanisoft Mini Kol" comprises an absorbing core made of fluff and super absorbents and an odour-absorbent textile layer of viscose coated with active carbon particles. Sanisoft Mini Kol further comprises a dispersion layer, also called acquisition layer, arranged between the absorbing core and a top sheet. The absorbing core has good characteristics for absorbing body fluids. Further, active carbon particles have proven to have good characteristics for absorbing odour, or odour particles. These articles have proven to be effective in absorbing body odours from body fluids for 3-5 hours. GB2308303 discloses a filter with two layers of activated carbon impregnated substrate 1 mm thick each and each containing 90g/m² of activated carbon. Said filter does not contain an absorbent article. US5154960 discloses an odour absorbent sheet material comprising an assembly of a thin flexible substrate sheet (fibrous sheet) having adhering on at least one side activated carbon at an amount of at least 120g/m² or 100g/m² as well as a cover sheet covering at least one side of the substrate sheet.

However, the applicant has observed a demand for even more efficient and longer-lasting odour-absorbent properties of sanitary articles. Further, the applicant has observed problems with odours that are not necessarily related to emission of body fluids or excrements. In more clear words, problems with uncontrolled emission of flatulence can be as annoying or embarrassing for the user as the problem with odours from body fluids.

Consequently, there is an interest for improved odour-absorbent sanitary articles with better odour-absorbent properties.

### Summary of invention

An object of embodiments of the present invention is to provide a sanitary article with improved odour-absorbent properties. Another object of embodiments is to provide a cost-efficient sanitary article for absorbing odour originating from flatulence problems.

When studying possible ways of improving the existing odour-absorbent sanitary articles, the applicant observed that the existing articles have a content of activated carbon of approximately 30 g/m². The applicant then tried with different increased content of activated carbon without seeing much difference in odour-absorbing performance. However, when the content of activated carbon in the layer was increased to at least 90 g/m², there was a significant increase in odour-absorbing performance.

Consequently, a layer is provided for a sanitary article for absorbing odour, the article being intended to be arranged in the crotch region of a human being, the layer comprising a textile fabric coated with at least 90 g/m² of activated carbon. As described above, a concentration of activated carbon of at least 90 g/m² on the textile fabric has proven to have a stronger and longer-lasting absorption of odour-generating particles compared to the prior art layers.

According to an aspect of the invention, a sanitary article for absorbing odour is provided comprising such an odour-absorbent layer coated with at least 90 g/m² of activated carbon as well as a non-woven cover enclosing the layer, Further, the sanitary article lacks an absorbent core. An important aspect of this sanitary article is that it does not comprise an absorbent core, comprising e.g. fluff and/or super absorbents, as the prior art sanitary articles for absorbing odour do. Such a sanitary article can for example be used for insertion into fluid-absorbing sanitary articles that do not have any odour-absorbent properties, for those articles to be able to absorb both body fluids and body odours. Adhesive areas are then used for attaching the article to the existing fluid-absorbing sanitary articles. Such a sanitary article may also be used solely as it is, especially for absorbing body odours occurring due to flatulence. Such an article would be much thinner than sanitary articles with absorbing cores and therefore much more pleasant for persons with embarrassing flatulence problems. Further, due to its much thinner form it is not at all discoverable from the outside.

The sanitary article of this aspect further comprises a folding indication running across the article from a first side of the article to a second side, opposite the first side. The folding indication facilitates folding of the sanitary article along the folding indication. The folding indication is applied so that approximately 50 % of the article is at one side of the folding indication and the other approximately 50 % of the article is on the other side of the folding indication. Such a product may be used for example for insertion between the buttocks of a user, in order to be closer to the anus thereby absorbing odour better, especially from flatulence, and still not be unpleasant for the user. The folding indication also makes the product more comfortable to wear between the buttocks compared to if no folding indication would have existed.

According to an embodiment of the sanitary article, the textile fabric of the odour-absorbent layer is principally made of polyester. As mentioned, the prior art article used viscose as the textile material onto which activated carbon particles were coated. By using polyester instead of viscose as the textile material for the odour-absorbent layer, a layer that is experienced as both softer and smoother for a user is achieved.

According to another embodiment, the textile fabric of the odour-absorbent layer is made of 85 - 90 % polyester and 10 - 15 % polyolefin. The polyolefin makes the layer capable of being hot moulded and welded which is an advantage when attaching the layer to a sanitary article. Polyolefin at the same time risks the layer to become stiff, i.e. not as soft and smooth as desired by the users. An apportionment of 85-90 % polyester and 10-15 % polyolefin has proven to give a soft and smooth product at the same time as the product has good moulding and welding properties.

According to another embodiment, the activated carbon of the odour-absorbent layer has a BET surface area of at least 1700m²/g. BET measures surface area based on gas adsorption. More specifically, it allows determination of the overall specific external and internal surface area of disperse (e.g. nano-powders) or porous solids by measuring the amount of physically adsorbed gas according to the Brunauer, Emmett and Teller (BET) method. Such an activated carbon type has proven to be more effective in absorbing odour particles as it has a larger surface area than standard activated carbon. For example, this type of activated carbon has proven to have a Carbon Tetrachloride, CTC, adsorption value of approximately 120 compared to a CTC adsorption value of 60-80 for the prior art absorbent articles. A standardized method for measuring the CTC value is described in ASTM D3467. This method measures the loading of carbon tetrachloride, weight percent on carbon, at concentrations close to saturation in the air. The method is basically a measure of the pore volume of the activated carbon and is primarily used as a quality assurance test for the production of activated carbon.

According to another embodiment, the odour-absorbent layer of the sanitary article has a thickness of 0.28 - 0.42 mm, preferably 0.30 - 0.40 mm. The remarkably thin odour-absorbent layer makes the article of this embodiment easier to fold along the folding indication than articles with thicker odour-absorbent layer.

According to another embodiment, the sanitary article further comprises one or more adhesive areas attached to a side of the non-woven cover facing away from the odour-absorbent layer, for attaching the sanitary article to a pair of underwear. The adhesive areas are used for attaching the article to e.g. a pair of underwear. Hereby, the article can be fixated close to the anus of the user for example in a folded position between the buttocks.

According to another embodiment, the sanitary article further comprising a release paper covering the one or more adhesive areas. The release paper makes an article as in the embodiment above easier to handle before it has been inserted into a pair of underwear. The release paper would be torn away before the article is inserted in the underwear.

According to another embodiment, the one or more adhesive areas comprise two adhesive areas arranged on different sides of the folding indication 52. By having two (or even more) separate adhesive areas arranged on different sides of the folding indication, the article is fixated better in the underwear than if only one adhesive area is used, or if two adhesive areas on the same side of the folding indication is used.

### Brief description of drawings

The invention is now described, by way of example, with reference to the accompanying drawings, in which:
Fig. 1A is a schematic top view of an odour-absorbent layer for a sanitary article.
Fig. 1B is a side view of the layer of fig. 1A.
Fig. 2 is a top view of a sanitary article for absorbing odour according to an embodiment.
Fig. 3 is a top view of a sanitary article according to another embodiment.
Fig. 4A is a top view of a liquid-absorbent sanitary article.
Fig. 4B is a cross-sectional side view of the liquid-absorbent sanitary article of fig. 4A, the cross-section being taken at section A-A in fig. 4A.
Fig. 5 is a top view of another liquid-absorbent sanitary article.
Fig. 6 is a flow chart illustrating a method for applying a liquid-absorbent sanitary article such as the article of fig. 5 into a pair of underwear of a male human being.
Fig. 7 is a flow chart illustrating a method for manufacturing an odour-absorbent layer as in figs. 1A and 1B.

### Description of embodiments

In the following, a detailed description of embodiments of an odour-absorbent layer is provided, as well as detailed descriptions of embodiments of sanitary articles having such an odour-absorbent layer. Further, an exemplary method for producing an odour-absorbent layer as well as a method for inserting a liquid-absorbent sanitary article comprising such an odour-absorbing layer is described.

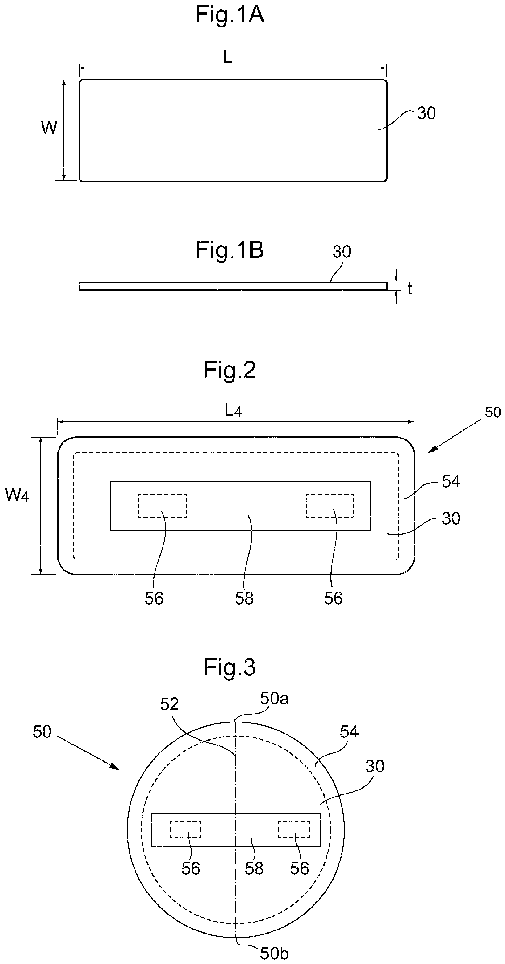

Figs. 1A and 1B show an odour-absorbent layer 30 for use in a sanitary article to be applied in the crotch region of a human being. The odour-absorbent layer 30 has a base of textile fabric coated with at least 90 g/m² of activated carbon. The odour-absorbent layer 30 has a width W and a length L. According to an embodiment, the length L may be between 150-190 mm and the width W may be between 35 and 45 mm. According to an embodiment, the layer 30 of activated carbon has a surface area, i.e. W*L, of at most 8000 mm², preferably at most 7000 mm². This is approximately 25 % smaller than the surface area of odour-absorbent layer of the prior art article Sanisoft Mini Kol. Thanks to the good odour-absorbing properties of the layer 30, it can be made smaller, and a possible increase in production cost due to the increase of concentration of activated carbon is at least partly recovered by the smaller layer size. Further, the odour-absorbent layer 30 may have a thickness of 0.28 - 0.42 mm, preferably 0.30 - 0.40 mm.

Fig. 2 describes an embodiment of a sanitary article 50 for absorbing odour that does not have any absorbent core for absorbing liquid. This article is instead designed for use for persons having flatulence problems not including liquid-emitting problems. Alternatively, the article may be used as a complement to a regular absorbing sanitary article not comprising an odour-absorbent layer. The sanitary article 50 of fig. 2 comprises an odour-absorbent layer 30 and a non-woven cover 54 enclosing the odour-absorbent layer 30. The non-woven cover 54 preferably extends further in length and width-direction than the odour-absorbent layer 30. In other words, the width W4 of the cover 54 may be larger than the width W of the odour-absorbent layer 30, and the length L4 of the cover 54 may be larger than the length L of the odour-absorbent layer 30. The exemplary sanitary article 50 of fig. 2 further comprises two adhesive areas 56 attached to a side of the non-woven cover 54 facing away from the layer 30, for attaching the sanitary article 50 to a sanitary pad or to a pair of underwear. The adhesive areas 56 are covered by a release paper 58 that is torn away to reveal the adhesive areas, before the sanitary article is attached to a sanitary pad or to a pair of underwear.

Fig. 3 shows an embodiment of another type of sanitary article 50 for absorbing odour that does not have any absorbent core for absorbing liquid. The sanitary article 50 of this type has a folding indication 52 that runs across the sanitary article 50 from a first side 50a of the article to an opposite second side 50b. This folding indication 52 is for facilitating folding of the sanitary article 50 along the folding indication 52. The embodiment shown in fig. 3 has a circular shape. However, other shapes may also apply, such as oval or a rectangular shape. The folding indication may be applied so that approximately 50 % of the article is at one side of the folding indication and the other approximately 50 % of the article is on the other side of the folding indication. The folding indication may run across the whole article from an edge of the first side 50a to an opposite edge of the second side 50b, or it may run across most of the article i.e. from a part of the first side 50a not being the edge to a part of the second side 50b not being the edge. The exemplary sanitary article 50 of fig. 3 further comprises two adhesive areas 56 attached to a side of the non-woven cover 54 facing away from the layer 30, for attaching the sanitary article 50 to a pair of underwear. The adhesive areas 56 are arranged on different sides of the folding indication 52. The adhesive areas are covered by a release paper 58 that is torn off before the sanitary article is attached to a pair of underwear.

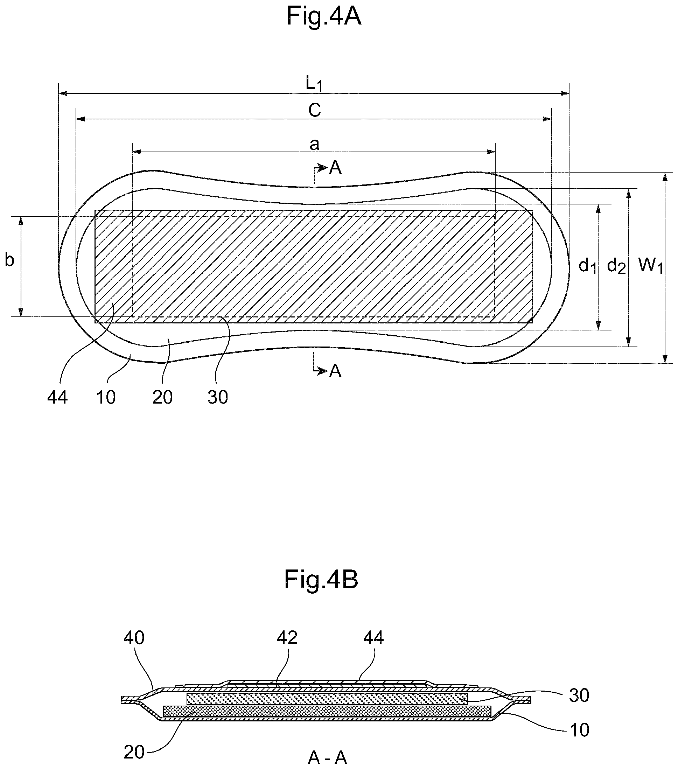

Figs. 4A and 4B describes a liquid-absorbent sanitary article comprising a liquid permeable top sheet 10, a liquid impermeable back sheet 40 and an absorbent core 20 arranged between the top sheet 10 and the back sheet 40. The absorbent core 20 comprises fluff and super absorbents for absorbing liquid. The top sheet 10 and the back sheet 40 are joined to each other at the ends of the liquid-absorbent article so that the absorbent core 20 is enclosed by the joined top sheet 10 and back sheet 40. The liquid-absorbent sanitary article further comprises an odour-absorbent layer 30 arranged between the absorbent core 20 and the back sheet 40. The odour-absorbent layer 30 comprises a textile fabric coated with at least 90 g/m² of activated carbon. Such a liquid-absorbent article would effectively absorb both body-emitted liquids and body-emitted odours. The liquid-absorbent sanitary article may further comprise an adhesive area 42 attached to the back sheet 40, on a side of the back sheet 40 facing away from the layer 30, for attaching the liquid-absorbent sanitary article to a pair of underwear. The adhesive area 42 may be covered by a release paper 44 that is to be torn away to reveal the adhesive area 42, before the liquid-absorbent sanitary article is attached to a pair of underwear.

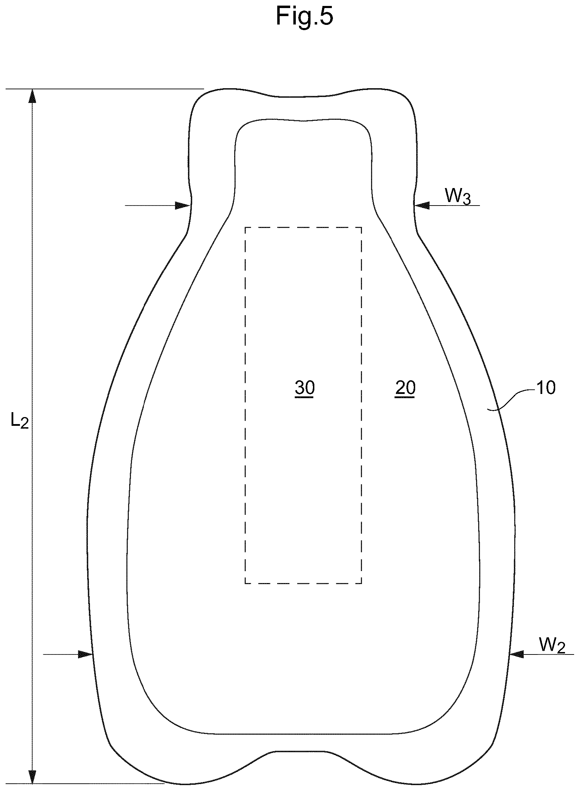

Fig. 5 is an alternative of a liquid-absorbent sanitary article as in fig. 4 especially adapted for absorbing anal leakage, i.e. excrements. The liquid-absorbent sanitary article may comprise similar layers as the article of fig. 4, however the shape of the article is adapted for receiving anal leakage by having a broader back part that can receive large amounts of excrements, and by its size prevents excrements to slip out on the sides of the article. The liquid-absorbent sanitary article of fig. 5 has a liquid permeable top sheet 10; a liquid impermeable back sheet 40 (see fig. 4B), an absorbent core 20 arranged between the top sheet 10 and the back sheet 40, the absorbent core comprising fluff and super absorbents for absorbing liquid, and, an odour-absorbent layer 30 comprising a textile fabric coated with at least 90 g/m² of activated carbon. The odour-absorbent layer 30 is arranged between the absorbent core 20 and the back sheet 40. The sanitary article further has a saddle shape and a longitudinal extension L₂ from its front part to its back part of 200 to 400 mm, a width W₃ at the front part of 50 to 90 mm, preferably 60 to 80 mm, and a width W₂ at a broadest part of the back part of 120 to 160 mm, preferably 130 to 150 mm. In the alternative shown in fig. 5, the absorbent core 20 has a shape similar to the shape of the whole article. However, other shapes may apply, for example the absorbent core may be smaller at the back part of the article. Further, the top sheet 10 and back sheet (not shown) may be joined at the ends of the article in order to enclose the absorbent core 20. The saddle-shaped article may further have at least one adhesive area attached to a side of the back sheet facing away from the absorbent core for attaching the saddle-shaped article to a sanitary pad or to a pair of underwear. The one or more adhesive area may be covered by one or more release paper. Such a comparatively broad back part enhances the possibilities to handle large emissions of excrements without risk of the excrements slip past the article and onto the underwear. Also, the article can be made rather thin as there is a comparatively large area for absorbing the excrements. A thin article is more pleasant for a user compared to a comparatively thicker article. Also, a comparatively thin article is more discrete for the user as it is more difficult for another person to see that a person wears a thin article than that a person wears a comparatively thicker article. Another purpose with the saddle-shaped form is that the product is adapted to be inserted behind the scrotum of a male user, in order not to put any pressure onto the male user's scrotum, as such pressure may be unpleasant for the user. For this reason, the male user is instructed to insert the article behind his scrotum so that the front end of the article ends just behind the scrotum when the article has been inserted into a pair of underwear.

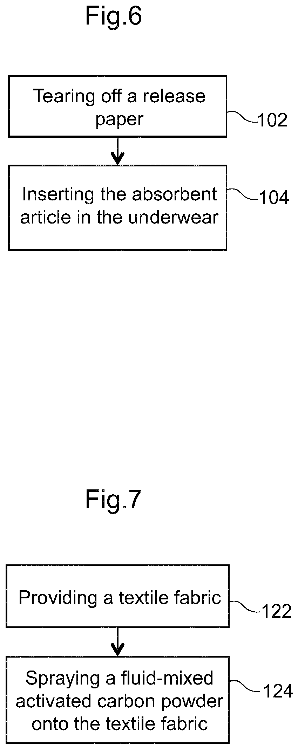

Fig. 6 describes a method for applying a liquid-absorbent sanitary article as in fig. 5 into a pair of underwear to be arranged onto a crotch region of a male human being so that the sanitary article of fig. 5 does not put pressure onto the scrotum of the male human being. The pair of underwear has a front part and a back part, the front part and the back part being joined by a crotch region and by an upper left part and an upper right part. Further, the front part, the back part, the crotch region, the upper left part and the upper right part define two leg openings mutually separated by the crotch region and a waist opening separated from the leg openings by the front part, the back part, the upper left part and the upper right part. The method comprises tearing 102 off a release paper arranged onto the one or more adhesive areas, and inserting 104 the liquid-absorbent article into the pair of underwear so that the longitudinal extension of the liquid-absorbent article extends from the front part of the underwear via the crotch region to the back part of the underwear. Further, the front part of the absorbent article extends up to a position of a scrotum of the male human being in the underwear and the back part extends into the back part of the underwear. Hereby, the male human being can easily insert the liquid-absorbing sanitary article into a pair of underwear and attach the article into his underwear using the adhesive areas in such a position that it is pleasant for him, avoiding unnecessary pressure on his scrotum.

Fig. 7 describes a method for manufacturing an odour-absorbent layer 30 (see fig. 1) for a sanitary article. The method comprises providing 122 a textile fabric, and spraying 124 an activated carbon powder mixed with a fluid, such as water, onto the textile fabric so that the textile fabric is coated with at least 90 g/m² of activated carbon. This method has proved to be successful in achieving such high concentration of activated carbon. Further, thanks to this type of spraying of the activated carbon onto the textile fabric instead of the physical transportation through a tank, the layer can be made thinner even though it has a higher concentration of activated carbon. Further, the spraying process results in a smooth layer compared to a layer produced according to the physical-transportation method, which becomes stiffer, the stiffer the more it is compressed. The fluid with which the active carbon powder is mixed before being sprayed onto the textile fabric may also comprise a surfactant and/or a binder. The purpose with the surfactant as well as with the binder is to make the textile fabric more acceptable to the activated carbon powder so that the activated carbon is more easily bound to the textile fabric. In such a manufacturing method, the carbon is in the form of a very fine powder, milled and classified to a size preferably below 10 microns. The spray process makes it possible to apply a coating to all fibres in the textile fabric as long as the layer is thinner than about 6mm. As the odour-absorbent layer 30 has a thickness far below 6 mm, all fibres in the textile fabric can be applied with the carbon powder. The carbon powder is preferably not impregnation-treated as impregnating to improve adsorption would introduce chemicals that may impact any medical device classification. Instead, a very highly activated carbon with a CTC-value 120 and BET surface area >1700m²/g may be used, which is more effective than standard activated carbon, which typically has a CTC-value of 60-80 and a BET surface area of 1000-1200m²/g.

## Claims

1. A sanitary article (50) for absorbing odour comprising:
an odour-absorbent layer (30) intended to be arranged in the crotch region of a human being, the odour-absorbent layer (30) comprising a textile fabric coated with at least 90 g/m² of activated carbon,
a non-woven cover (54) enclosing the odour-absorbent layer (30),
wherein the sanitary article (50) lacks an absorbent core, **characterized in that** the sanitary article further comprises a folding indication (52) running across the sanitary article (50) from a first side (50a) of the article to a second side (50b), opposite the first side, the folding indication (52) facilitating folding of the sanitary article (50) along the folding indication (52), wherein the folding indication is applied so that approximately 50 % of the article is at one side of the folding indication and the other approximately 50 % of the article is on the other side of the folding indication.

2. Sanitary article (50) according to claim 1, wherein the textile fabric of the odour-absorbent layer is principally made of polyester.

3. Sanitary article (50) according to claim 2, wherein the textile fabric of the odour-absorbent layer is made of 85 - 90 % polyester and 10 - 15 % polyolefin.

4. Sanitary article (50) according to any of the preceding claims, wherein the activated carbon has a BET surface area of at least 1700m²/g.

5. Sanitary article (50) according to any of the preceding claims, wherein the odour-absorbent layer has a thickness of 0.28 - 0.42 mm, preferably 0.30 - 0.40 mm.

6. Sanitary article according to any of the preceding claims, further comprising one or more adhesive areas (56) attached to a side of the non-woven cover (54) facing away from the layer (30), for attaching the sanitary article (50) to a pair of underwear.

7. Sanitary article according to claim 6, further comprising a release paper (58) covering the one or more adhesive areas (56).

8. Sanitary article according to claim 6 or 7, the one or more adhesive areas (56) comprising two adhesive areas arranged on different sides of the folding indication (52).

## Patentansprüche

1. Hygieneartikel (50) zum Absorbieren von Geruch, umfassend:
eine Geruch absorbierende Schicht (30), die dazu vorgesehen ist, im Schrittbereich eines Menschen angeordnet zu werden, wobei die Geruch absorbierende Schicht (30) einen Textilstoff umfasst, der mit mindestens 90 g/m² Aktivkohle beschichtet ist,
eine Vliesabdeckung (54), die die Geruch absorbierende Schicht (30)umschließt,
wobei dem Hygieneartikel (50) ein absorptionsfähiger Kern fehlt, **dadurch gekennzeichnet, dass** der Hygieneartikel ferner einen Falthinweis (52) umfasst, der über den Hygieneartikel (50) von einer ersten Seite (50a) des Artikels zu einer zweiten Seite (50b) gegenüber der ersten Seite verläuft, wobei der Falthinweis (52) das Falten des Hygieneartikels (50) entlang des Falthinweises (52) ermöglicht, wobei der Falthinweis derart aufgebracht ist, dass sich ungefähr 50 % des Artikels auf einer Seite des Falthinweises befinden und sich die anderen ungefähr 50 % des Artikels auf der anderen Seite des Falthinweises befinden.

2. Hygieneartikel (50) nach Anspruch 1, wobei der Textilstoff der Geruch absorbierenden Schicht vorwiegend aus Polyester besteht.

3. Hygieneartikel (50) nach Anspruch 2, wobei der Textilstoff der Geruch absorbierenden Schicht zu 85-90 % aus Polyester und zu 10-15 % aus Polyolefin besteht.

4. Hygieneartikel (50) nach einem der vorstehenden Ansprüche, wobei die Aktivkohle eine BET-Oberfläche von mindestens 1700 m²/g aufweist.

5. Hygieneartikel (50) nach einem der vorstehenden Ansprüche, wobei die Geruch absorbierende Schicht eine Dicke von 0,28-0,42 mm, vorzugsweise von 0,30-0,40 mm aufweist.

6. Hygieneartikel nach einem der vorstehenden Ansprüche, ferner umfassend eine oder mehrere Klebeflächen (56), die zum Befestigen des Hygieneartikels (50) an der Unterwäsche an einer von der Schicht (30) abgewandten Seite der Vliesabdeckung (54) befestigt sind.

7. Hygieneartikel nach Anspruch 6, ferner umfassend ein Abziehpapier (58), das die eine oder die mehreren Klebeflächen (56) abdeckt.

8. Hygieneartikel nach Anspruch 6 oder 7, wobei die eine oder die mehreren Klebeflächen (56) zwei Klebeflächen umfassen, die an unterschiedlichen Seiten des Falthinweises (52) angeordnet sind.

## Revendications

1. Article d'hygiène (50) pour absorber les odeurs comprenant :
une couche d'absorption des odeurs (30) destinée à être disposée dans la région d'entrejambe d'un être humain, la couche d'absorption des odeurs (30) comprenant un tissu textile revêtu d'au moins 90 g/m² de charbon actif,
un revêtement non tissé (54) enfermant la couche d'absorption des odeurs (30),
l'article d'hygiène (50) ne comportant pas de cœur absorbant, **caractérisé en ce que** l'article d'hygiène comprend en outre une indication de pliage (52) parcourant l'étendue de l'article d'hygiène (50) depuis un premier côté (50a) de l'article vers un second côté (50b), en regard du premier côté, l'indication de pliage (52) facilitant le pliage de l'article d'hygiène (50) le long de l'indication de pliage (52), dans lequel l'indication de pliage est appliquée de sorte que 50 % environ de l'article se situent au niveau d'un côté de l'indication de pliage et que les autres 50 % environ de l'article se situent sur l'autre côté de l'indication de pliage.

2. Article d'hygiène (50) selon la revendication 1, dans lequel le tissu textile de la couche d'absorption des odeurs est principalement en polyester.

3. Article d'hygiène (50) selon la revendication 2, dans lequel le tissu textile de la couche d'absorption des odeurs est constitué de 85 à 90 % de polyester et de 10 à 15 % de polyoléfine.

4. Article d'hygiène (50) selon l'une quelconque des revendications précédentes, dans lequel le charbon activé a une surface spécifique BET d'au moins 1 700 m²/g.

5. Article d'hygiène (50) selon l'une quelconque des revendications précédentes, dans lequel la couche d'absorption des odeurs a une épaisseur de 0,28 à 0,42 mm, de préférence de 0,30 à 0,40 mm.

6. Article d'hygiène selon l'une quelconque des revendications précédentes, comprenant en outre une ou plusieurs zones adhésives (56) fixées à un côté du revêtement non tissé (54) à l'opposé de la couche (30), pour fixer l'article d'hygiène (50) à des sous-vêtements.

7. Article d'hygiène selon la revendication 6, comprenant en outre un papier de protection (58) recouvrant la ou les zones adhésives (56).

8. Article d'hygiène selon la revendication 6 ou 7, la ou les zones adhésives (56) comprenant deux zones adhésives disposées sur différents côtés de l'indication de pliage (52).
